# EUROPÄISCHE PATENTSCHRIFT

(11) **EP 1 106 199 B1**
(45) Veröffentlichungstag und Bekanntmachung des Hinweises auf die Patenterteilung: **27.07.2005**
(21) Anmeldenummer: 00124007.6
(22) Anmeldetag: 04.11.2000
(51) Int. Cl.: A61M 25/00, A61M 25/06, A61M 5/32

(54) **Kurzkatheter**
Microcatheter
Microcathéter

(30) Priorität: 01.12.1999 DE 29921084 U
(43) Veröffentlichungstag der Anmeldung: 13.06.2001
(73) Patentinhaber: B. BRAUN MELSUNGEN AG, 34212 Melsungen (DE)
(72) Erfinder: Woehr, Kevin, 34587 Felsberg (DE)
(74) Vertreter: Klingseisen, Franz

(56) Entgegenhaltungen:
- WO-A-99/08742

## Beschreibung

Die Erfindung betrifft einen Kurzkatheter nach dem Oberbegriff des Anspruchs 1.

Bei einem solchen Kurzkatheter wird die in das Katheterrohr eingeschobene Nadel nach dem Herausziehen durch einen Nadelschutz unbrauchbar gemacht.

Kurzkatheter werden auch als Venenkatheter oder IV-Katheter bezeichnet. Sie weisen ein flexibles Katheterrohr auf, das an einem Ende mit einem Katheteransatz versehen ist. In das Katheterrohr ist eine hohle Nadel eingeschoben, die am distalen Ende eine schneidende Nadelspitze und am proximalen Ende einen Nadelansatz aufweist. Mit der Nadelspitze wird die Nadel mit dem sie umgebenden Katheterrohr in den Körper des Patienten eingeführt. Nachdem die Spitze des Katheterrohres in eine Vene eingedrungen ist, wird die Nadel zurückgezogen.

Aus WO 99/08742 ist ein Kurzkatheter nach dem Oberbegriff des Anspruchs 1 bekannt, bei dem die Nadel mit einem Nadelschutz versehen ist. Der Nadelschutz besteht aus einer metallischen elastischen Klemme, die in dem Hohlraum des Katheteransatzes enthalten ist und Löcher für den Durchtritt für die Nadel aufweist. Der Nadelschutz drückt mit einem Hakenteil seitlich gegen die Nadel. Wenn beim Zurückziehen der Nadel die Nadelspitze das Hakenteil passiert, schnappt das Hakenteil über die Nadelspitze über, so dass diese von dem Hakenteil übergriffen wird und nicht mehr zugänglich ist. Dadurch werden Verletzungen von Personen durch die Nadelspitze verhindert. Insbesondere wird auch die Kontaminationsgefahr durch an der Nadel haftende Keime, die beim erstmaligen Gebrauch auf die Nadel übertragen wurden, verringert. Durch den Nadelschutz ist sichergestellt, dass die Nadel nur einmal gebraucht werden kann, so dass nicht eine kontaminierte Nadel bei einem anderen Patienten wiederbenutzt wird. Um das Abgleiten des Nadelschutzes über das distale Nadelende hinaus zu verhindern, kann an der Nadel ein entsprechendes Rastmittel in Form einer Kerbe oder einer Unrundheit vorgesehen sein, welches einen distalen Anschlag zur Begrenzung der Bewegung des Nadelschutzes bildet.

Der vorliegenden Erfindung liegt die Aufgabe zugrunde, einen Kurzkatheter mit Nadelschutz zu schaffen, bei dem der Nadelschutz in seiner die Nadelspitze überdeckenden Position infolge von Klemmwirkung einen festen Halt hat.

Der erfindungsgemäße Kurzkatheter weist die im Anspruch 1 angegebenen Merkmale auf. Hiernach ist der Nadelschutz in dem Katheteransatz in axialer Richtung vorgespannt, wobei seine Enden gegen axiales Ausdehnen fixiert sind. Im vorgespannten Zustand kann die Nadel leicht durch die Löcher des Nadelschutzes hindurchgeschoben werden, weil die Löcher dann so orientiert sind, dass die Nadel leicht verschoben werden kann. Im Auslösefall, wenn also die Nadelspitze das Hakenteil passiert hat, schnappt das Hakenteil über die Nadelspitze über, wodurch die axiale Spannung des Federelements aufgehoben wird. Das Federelement wird dadurch in seine gestreckte Ursprungsform zurückversetzt. Dadurch werden die Löcher, die zuvor die Nadel mit geringem Spiel umgeben hatten, plötzlich in einer Richtung enger, wodurch das Federelement sich an der Nadel festkrallt. Durch das Aufheben der axialen Vorspannung und das Zurückfedern des Federelements wird eine feste Klemmung der Nadel an den Lochrändern des Federelements bewirkt. Dadurch kann in vielen Fällen auf Rast- oder Blockiermittel an der Nadel verzichtet werden, so dass die Nadel gegenüber herkömmlichen Nadeln keine Veränderung erfahren muß.

Damit der Nadelschutz im axial komprimierten Zustand in dem Innenraum des Katheteransatzes untergebracht werden kann, muß im Katheteransatz ein Haltemittel vorgesehen sein, welches einen Anschlag für das distale Ende des Nadelschutzes, nämlich das Hakenteil, bildet. Ein solches Haltemittel besteht vorzugsweise aus einem Metallteil, welches in das Katheterrohr ragt und dieses in dem Katheteransatz abstützt. Ein solches Metallteil ist als interne Katheterabstützung üblicherweise in einem Katheteransatz enthalten. Das Metallteil kann auf einfache Weise modifiziert werden, um eine Anschlagschulter für den Nadelschutz zu bilden. Diese Anschlagschulter kann aus einer Stirnwand bestehen, die am Öffnungsende eines Trichters des Metallteils angeformt ist.

Vorzugsweise besteht ein proximales Haltemittel aus einer Endkante des Nadelschutzes, welche in die Wand des Katheteransatzes hineinragt. Diese Endkante kann angeschärft sein und sich bei der Montage des Nadelschutzes in die Wand des Katheteransatzes eingraben.

Im folgenden wird unter Bezugnahme auf die Zeichnungen ein Ausführungsbeispiel der Erfindung näher erläutert.

Es zeigen:
- Fig. 1: einen Längsschnitt durch den Kurzkatheter,
- Fig. 2: einen Längsschnitt nach dem Einsetzen der Nadel, wobei der Kurzkatheter im anwendungsbereiten Zustand ist,
- Fig. 3: einen Schnitt entlang der Linie III-III von Fig. 2,
- Fig. 4: einen Längsschnitt während des Herausziehens der Nadel und
- Fig. 5: einen Schnitt entlang der Linie V-V von Fig. 4.
Der dargestellte Kurzkatheter weist ein flexibles Katheterrohr 10 aus elastischem Kunststoff auf, das am proximalen Ende mit einem Katheteransatz 11 versehen ist. Der Katheteransatz 11 besteht aus einem langgestreckten hohlen Kunststoffteil, das einen leicht konischen Innenraum 12 hat und am proximalen Ende mit einem Luer-Anschluß 13 versehen ist. Der distale Endbereich 14 ist rohrförmig. Durch ihn ragt das Katheterrohr 10 bis zum Innenraum 12. In das proximale Ende des Katheterrohrs 10 ist ein rohrförmiges Metallteil 15 eingeschoben, welches das Katheterrohr aufweitet und gegen die Wand des rohrförmigen Bereichs 14 drückt. Am proximalen Öffnungsende des Metallteils 15 ist das Metallteil als Trichter 16 aufgeweitet. Dem Öffnungsende des Trichters 16 ist eine radial nach innen weisende Stirnwand 17 angeformt.

In das Katheterrohr 10 wird eine hohle Nadel 18 eingeschoben, die am distalen Ende eine schneidende Nadelspitze 19 aufweist. Die Nadel 18 besteht aus Stahl. Sie ist am rückwärtigen Ende mit einem Nadelansatz 20 aus Kunststoff verbunden. Der Nadelansatz 20 stößt gegen das proximale Ende des Katheteransatzes 11 und er ragt dann mit einem kegelstumpfförmigen Vorsprung 21, der locker in den Katheteransatz 11 hineinpaßt, in den Innenraum 12.

Der Innenraum 12 enthält den Nadelschutz 22, der aus einem Federelement aus gebogenem Federstahlblech besteht. Der Nadelschutz ist generell Z-förmig gebogen, wobei ein rückwärtiger Schenkel 23, ein mittlerer Schenkel 24 und ein vorderer Schenkel 25 aufeinander folgen. Dem vorderen Schenkel 25 ist ein Hakenteil 26 angeformt, welches um mehr als 90° umgebogen ist. In dem rückwärtigen Schenkel 23 und dem mittleren Schenkel 24 sind jeweils ein Loch 27 bzw. 28 vorhanden.

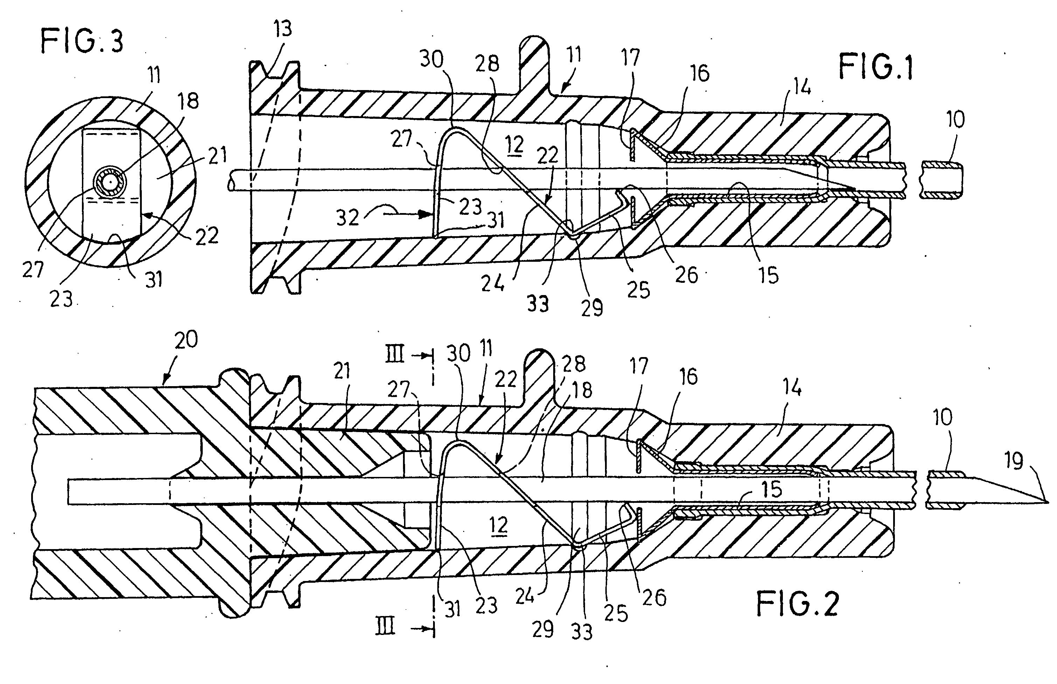

Der Nadelschutz 22 wird gemäß Fig. 1 auf der Nadel 18 sitzend in den Katheteransatz eingesetzt, wobei er mit dem Hakenteil 26 gegen die Stirnwand 17 des Metallteils 16 gesetzt wird. Dann wird mit einem (nicht dargestellten) Werkzeug, das vom proximalen Ende her in den Innenraum 12 eingeführt wird, gemäß Pfeil 32 axialer Druck ausgeübt, wobei der Nadelschutz 22 in axialer Richtung komprimiert wird. Die axiale Richtung ist an der Ausrichtung der Achse des Katheterrohres 10 orientiert. Bei der axialen Kompression wird ein zwischen den Abschnitten 24 und 25 gebildetes vorderes Knie 33 des Nadelschutzes in eine innere umlaufende Nut 29 des Innenraums gedrückt. Ein entgegengesetztes Knie 30 verbindet die Abschnitte 23 und 24.

In diesem zusammengedrückten Zustand des Nadelschutzes 22 sind die Löcher 27,28 in axialer Projektion kreisrund. Dies bedeutet, dass das Loch 28 im schrägen mittleren Schenkel 24 oval ist und in Längsrichtung eine größere Ausdehnung hat als in Querrichtung.

Beim axialen Zusammendrücken des Nadelschutzes 22 wird die Endkante 31 des Abschnitts 23 in die Wand des Katheteransatzes 11 eingedrückt. Dadurch verkrallt sich das rückwärtige Ende des Nadelschutzes in der Wand, während das vordere Ende des Nadelschutzes an der Stirnwand 17 abgestützt ist. In diesem Zustand kann die Nadel 18 vom proximalen Ende her eingeschoben werden, wobei sie durch die Löcher 27 und 28 mit Spiel hindurchgeht und widerstandslos vorgeschoben werden kann. Das Hakenteil 26 wird von der Nadel 18 radial nach außen verschoben, so dass es nach dem weiteren Vorschieben der Nadel 18 seitlich gegen die Nadel drückt.

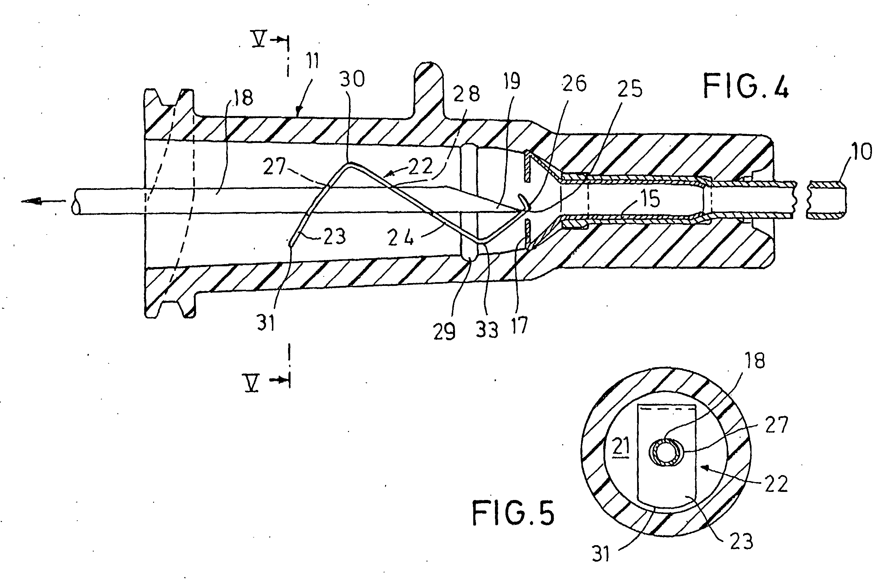

Die Fign. 4 und 5 zeigen den Zustand des Kurzkatheters während des Zurückziehens der Nadel 18 aus dem Katheterrohr 10 und dem Katheteransatz 11. Während des Zurückziehens der Nadel 18 schnappt das Hakenteil 26, das zuvor seitlich gegen die Nadel gedrückt hatte, über die Nadelspitze 19 über. Dabei entspannt sich der zuvor axial zusammengedrückte Nadelschutz und die Endkante 31 löst sich aus ihrer Verankerung in dem Katheteransatz. Der federnde Nadelschutz 22 streckt sich dadurch in axialer Richtung und die scharfen Kanten der Löcher 27,28 graben sich in die Nadel 18 ein. Wie aus Fig. 5 erkennbar ist, wird das Loch 27 in axialer Projektion zu einer Ellipse. In gleicher Weise wird das Loch 28 in axialer Projektion zu einer Ellipse. Sobald also der Nadelschutz dadurch ausgelöst wurde, dass die Nadelspitze 19 das Hakenteil 26 passiert, schnappt das Hakenteil 26 über die Nadelspitze über und der Nadelschutz verkrallt sich an der Nadel. Das Hakenteil 26 schirmt nun die Nadelspitze ab und blockiert eine distale Bewegung der Nadelspitze in bezug auf den Nadelschutz. Dadurch, dass der Nadelschutz axial expandiert und seine Abstützung an dem Katheteransatz aufgibt, kann er im Katheteransatz frei bewegt werden. Nach dem Auslösen des Nadelschutzes greift dieser mit zwei Löchern 27,28 an insgesamt vier Angriffspunkten an der Nadel an. Die Nadel 18 ist eine zylindrische Rundnadel ohne jegliche Einkerbungen, Vorsprünge oder Unrundheiten.

## Patentansprüche

1. Kurzkatheter mit
einem Katheterrohr (10), das am proximalen Ende einen Katheteransatz (11) aufweist,
einer durch das Katheterrohr (10) steckbaren hohlen Nadel (18), die am proximalen Ende einen Nadelansatz (20) aufweist,
wobei der Nadelansatz (20) einen Vorsprung (21) hat, welcher in den Innenraum (12) des Katheteransatzes (11) hineinragt,
und mit einem im Innenraum (12) des Katheteransatzes (11) auf der Nadel (18) angeordneten Nadelschutz (22) aus einem Federelement, das Löcher (27,28) für den Durchgang der Nadel (18) und am distalen Ende ein Hakenteil (26) aufweist, und
wobei der Nadelschutz (22) in dem Katheteransatz (11) in axial gespanntem Zustand enthalten ist, wobei seine Enden gegen axiales Ausdehnen blockiert sind, **dadurch gekennzeichnet,**
**dass** die Löcher (27, 28) oval sind, derart, dass bei axial zusammengedrücktem Nadelschutz (22) die Löcher in axialer Projektion rund sind und die Nadel (18) mit Spiel umgeben.

2. Kurzkatheter nach Anspruch 1, **dadurch gekennzeichnet, dass** ein distales Blockierteil aus einem Metallteil (15) besteht, welches in das Katheterrohr (10) ragt und dieses in dem Katheteransatz (11) abstützt.

3. Kurzkatheter nach Anspruch 2, **dadurch gekennzeichnet, dass** das Metallteil (15) am Öffnungsende eines Trichters (16) eine nach innen weisende Stirnwand (17) aufweist.

4. Kurzkatheter nach einem der Ansprüche 1-4, **dadurch gekennzeichnet, dass** ein proximales Blockiermittel aus einer Endkante (31) des Nadelschutzes (22) besteht, welche in die Wand des Katheteransatz (11) hineinragt.

5. Kurzkatheter nach einem der Ansprüche 1-4, **dadurch gekennzeichnet, dass** das Hakenteil (26) gegen die Nadel (18) drückt und beim Passieren der Nadelspitze (19) derart über die Nadelspitze schnappt, dass der Nadelschutz (22) sich entspannt und mit Kanten seiner Löcher (27,28) seitlich an der Nadel (18) angreift.

## Claims

1. Short catheter comprising
a catheter tube (10) having a hub (11) on the proximal end, a hollow needle (18) which can be inserted into the catheter tube (10) and has a needle hub (20) on the proximal end,
wherein the needle hub (20) has a projection (21) projecting into the inner chamber (12) of the catheter hub (11),
and a needle guard (22) consisting of a spring element, which needle guard is arranged on the needle (18) in the inner chamber (12) of the catheter hub (11) and has holes (27, 28) for the passage of the needle (18) and a hook member (26) on the distal end, and
wherein the needle guard (22) is positioned in the catheter hub (11) in axially biased state, wherein its ends are blocked against axial movement,
**characterized in that**
the holes (27, 28) are oval, such that in an axially compressed needle guard (22) the holes are round in axial projection and surround the needle (18) with clearance.

2. Short catheter according to claim 1,
**characterized in that** a distal blocking portion consists of a metal member (15) which projects into the catheter tube (10) and supports this in the catheter hub (11).

3. Short catheter according to claim 2,
**characterized in that** the metal member (15) on the opening end of a funnel (16) has an end wall (17) facing inwardly.

4. Short catheter according to one of the claims 1 to 4,
**characterized in that** a proximal blocking means consists of an end edge (31) of the needle guard (22) which projects into the wall of the catheter hub (11).

5. Short catheter according to one of the claims 1 to 4,
**characterized in that** the hook member (26) presses against the needle (18) and when passing the needle tip (19) snaps over the needle tip such that the needle guard (22) relaxes and engages with edges of its holes (27, 28) laterally on the needle (18).

## Revendications

1. Cathéter court avec
un tube de cathéter (10) montrant à l'extrémité proximale un embout de cathéter (11),
une aiguille creuse (18) passant au travers du tube de cathéter (10) montrant à l'extrémité proximale un embout d'aiguille (20),
dont l'embout d'aiguille (20) a une saillie (21) pénétrant dans l'intérieur (12) de l'embout de cathéter (11),
et avec une protection d'aiguille (22) consistant en un élément ressort se trouvant à l'intérieur (12) de l'embout de cathéter (11) sur l'aiguille (18) possédant des perforations (27,28) pour le passage de l'aiguille (18) et un élément de crochet (26) situé à l'extrémité distale, et
dont la protection d'aiguille (22) se trouve dans l'embout de cathéter (11) à l'état de tension axiale dont ses extrémités sont bloquées pour éviter une dilatation axiale,
**caractérisé en ce que**
les perforations (27,28) sont ovales de telle sorte qu'en serrant la protection d'aiguille (22) axialement les perforations s'arrondissent en projection axiale et l'aiguille (18) prend du jeu.

2. Cathéter court selon la revendication 1, **caractérisé en ce qu'**un élément de blocage distal se compose d'une pièce métallique (15) pénétrant dans le tube de cathéter (10) et l'appuyant à l'embout de cathéter (11).

3. Cathéter court selon la revendication 2, **caractérisé en ce que** la pièce métallique (15) possède à l'extrémité de l'ouverture d'un entonnoir (16) une paroi frontale (17) vers l'intérieur.

4. Cathéter court selon l'une quelconque des revendications 1 à 3, **caractérisé en ce qu'**un élément de blocage proximal se compose à l'extrémité d'un bord (31) de la protection de l'aiguille (22) pénétrant dans la paroi de l'embout de cathéter (11).

5. Cathéter court selon l'une quelconque des revendications 1 à 4, **caractérisé en ce que** l'élément de crochet (26) pousse contre l'aiguille (18) et en passant la pointe d'aiguille (19) il la dépasse de telle manière que la protection de l'aiguille (22) se détend et s'accroche de côté à l'aiguille (18) avec les bords de ses perforations (27, 28).
